# EUROPÄISCHE PATENTSCHRIFT

(11) **EP 1 313 457 B1**
(45) Veröffentlichungstag und Bekanntmachung des Hinweises auf die Patenterteilung: **14.06.2006**
(21) Anmeldenummer: 01960676.3
(22) Anmeldetag: 18.08.2001
(51) Int. Cl.: A61K 9/70, A61K 31/135, A61P 25/02, A61P 25/18, A61P 25/22

(54) **TRANSDERMALES THERAPEUTISCHES SYSTEM ZUR ABGABE VON VENLAFAXIN**
TRANSDERMAL THERAPEUTIC SYSTEM FOR RELEASING VENLAFAXINE
SYSTEME THERAPEUTIQUE TRANSDERMIQUE DESTINE A LA DELIVRANCE DE LA VENLAFAXINE

(30) Priorität: 30.08.2000 DE 10042412
(43) Veröffentlichungstag der Anmeldung: 28.05.2003
(73) Patentinhaber: LTS LOHMANN Therapie-Systeme AG, 56626 Andernach (DE)
(72) Erfinder: SELZER, Thorsten, 56564 Neuwied (DE)
(74) Vertreter: Flaccus, Rolf-Dieter
(86) Internationale Anmeldenummer: PCT/EP2001/009531
(87) Internationale Veröffentlichungsnummer: WO 2002/017889

(56) Entgegenhaltungen:
- WO-A-00/00120
- WO-A-00/59851

## Beschreibung

Die vorliegende Erfindung betrifft transdermale therapeutische Systeme mit dem Wirkstoff Venlafaxin, welche die Verabreichung dieses Wirkstoffs über die Haut ermöglichen. Die Erfindung umfaßt ferner die therapeutische Verwendung derartiger Systeme bei verschiedenen medizinischen Indikationen.

Venlafaxin ist ein Serotonin- und Noradrenalin-Wiederaufnahme-Hemmer und wird zur Therapie und Prophylaxe von Depressionen und Angstzuständen eingesetzt.

Die orale Applikation von Venlafaxin ist allerdings mit einer Reihe von Nachteilen verbunden, die durch die Pharmakokinetik bedingt sind. Nach erfolgter oraler Verabreichung treten in der Anfangsphase, d. h. nach ca. 2 bis 4 h, "Peak-Plasma-Spitzen" auf, d. h. die Venlafaxin-Plasmakonzentration erreicht kurzzeitig relativ hohe Werte. Dies kann bei einer üblichen Einzeldosis von 25 mg zu unerwünschten Nebenwirkungen führen.
Andererseits wird Venlafaxin relativ schnell metabolisiert und ausgeschieden. Die Eliminationshalbwertszeit liegt im Bereich von 3 bis 5 h und ist somit recht kurz. Dies führt zu einem relativ raschen Nachlassen der Wirkung, so daß pro Tag 2 bis 3 aufeinanderfolgende Einzelapplikationen notwendig werden. Diese Nachteile sind insbesondere vor dem Hintergrund einer Dauertherapie, beispielsweise bei Depressionen, zu berücksichtigen.

Transdermale therapeutische Systeme (TTS) sind Darreichungsformen, die auf die Haut appliziert werden und einen Wirkstoff an die Haut abgeben, wobei dieser Wirkstoff dadurch systemisch verfügbar gemacht wird.
TTS bestehen nach dem Stand der Technik aus einer arzneistoffundurchlässigen Trägerschicht (auch Rückschicht genannt), einer arzneistoffhaltigen Reservoirschicht sowie einer Haftklebeschicht zur Befestigung auf der Haut. Die letztgenannte Schicht kann auch mit der arzneistoffhaltigen Schicht identisch sein. Außerdem weisen TTS in der Regel eine vor der Applikation zu entfernende, ebenfalls wirkstoffundurchlässige Rückschicht auf. Zusätzlich können noch andere Komponenten vorhanden sein, wie z. B. eine die Wirkstoffabgabe begrenzende Steuermembran. Die arzneistoffhaltige Reservoirschicht besteht meist aus polymeren Grundsubstanzen; sie kann ferner verschiedene Hilfs- oder Zusatzstoffe enthalten.

Aufgabe der vorliegenden Erfindung war es, eine Darreichungsform für den Wirkstoff Venlafaxin bereitzustellen, bei deren Anwendung die Nachteile vermieden werden, die mit der oralen Verabreichung von Venlafaxin einher gehen, wie oben beschrieben.
Ferner sollte diese Darreichungsform einen genügend hohen Wirkstoff-Flux in vivo ermöglichen.
Des weiteren ist zu fordern, daß eine solche Darreichungsform mittels gängiger Herstellungsverfahren kostengünstig produziert werden kann.

Überraschenderweise wird diese Aufgabe gelöst durch ein transdermales therapeutisches System in Pflasterform gemäß Anspruch 1, sowie durch die in den Unteransprüchen beschriebenen besonderen Ausführungsformen.

Folglich umfaßt die Erfindung TTS in Pflasterform zur Verabreichung des Wirkstoffs Venlafaxin, welche eine wirkstoffundurchlässige Rückschicht, ein damit verbundenes, den Wirkstoff Venlafaxin enthaltendes Wirkstoffreservoir, eine hautseitige haftklebende Schicht und eine vor der Applikation ablösbare wirkstoffundurchlässige Schutzschicht aufweisen.

Die erfindungsgemäßen TTS ermöglichen über einen verlängerten Applikations-Zeitraum eine konstante Abgabe des Wirkstoffs Venlafaxin an und durch die Haut, wodurch dieser Wirkstoff systemisch verfügbar wird. Auf diese Weise kann die relativ rasche Elimination von Venlafaxin durch ständiges Nachliefern aus dem Wirkstoffreservoir des TTS kompensiert werden, und der therapeutische Wert der Arzneistoffverabreichung wird erhöht.
Dies ist insbesondere bei der Dauertherapie von Depressionen vorteilhaft. Gegenüber oralen Darreichungsformen läßt sich eine fortgesetzte therapeutische Wirkung mit einer relativ niedrigen Applikationsfrequenz erzielen.
Weitere Vorteile beruhen darauf, daß bei der transdermalen Verabreichung die Metabolisierung des Wirkstoffs während der ersten Darm-Leber-Passage ("First-Pass"-Effekt) verhindert wird und damit die Halbwertszeit erhöht wird. Außerdem können auf diese Weise Probleme wie gastrointestinale Intoleranz oder unzureichende enterale Absorption vermieden werden.

Die erfindungsgemäßen TTS können sowohl in Form von Matrix-Systemen als auch in Form von Beutel-Reservoir- bzw. Membransystemen hergestellt werden.
Der Begriff "Matrixsysteme" schließt nicht nur solche Systeme ein, bei denen der Wirkstoff in einer schichtförmigen Kunstharz- oder Kunststoffmatrix gelöst ist und aus dieser abgegeben wird, sondern auch solche, in denen der Wirkstoff an Fasermaterial, wie z. B. Baumwollgewebe oder Baumwollvlies adsorbiert ist. Dieses Fasermaterial kann in einer Kunststoff- oder Kunstharzmatrix eingebettet sein.

Grundsätzlich können für die Herstellung des Wirkstoffreservoirs bzw. der Wirkstoffmatrix eine Vielzahl von Polymeren, Harzen und Zusatzstoffen eingesetzt werden, sofern die mit der Haut in Berührung kommenden Stoffe hautverträglich sind und solange die damit hergestellte Formulierung in der Lage ist, den Wirkstoff Venlafaxin an die Haut abzugeben.

Das Wirkstoffreservoir der erfindungsgemäßen TTS kann ferner verschiedene Hilfs- oder Zusatzstoffe enthalten, beispielsweise aus der Gruppe der Lösungsvermittler, Lösungsmittel, Weichmacher, Permeationsverbesserer, pH-Regulatoren, Antioxidantien und Konservierungmittel.

Bei der Herstellung der erfindungsgemäßen TTS kann im einfachsten Fall so vorgegangen werden, daß Venlafaxin in einer Lösung von Matrix-Grundpolymeren grob, kolloidal oder molekular dispergiert wird und die Mischung auf eine geeignete Unterlage, beispielsweise eine mit einer Silikonschicht versehene thermoplastische Folie, beschichtet wird. Nach Trocknen und Abdampfen der Lösemittelanteile wird die wirkstoffhaltige Matrixschicht mit einer weiteren Folie abgedeckt, welche die spätere Rückschicht des TTS darstellt. Durch Stanzen flächiger Gebilde in der gewünschten geometrischen Form und Größe werden aus einem solchen Laminat TTS hergestellt.

Geeignete Grundpolymere für die Wirkstoffmatrix und die haftklebende Schicht sind Polyacrylate, Poly(meth)acrylate, Polyacrylsäure, Cellulose-Derivate, insbesondere Methyl-und Ethylcellulosen, Isobutylen, Ethylen-Vinylacetat, natürliche und synthetische Kautschuke wie Styrol-Dien-Copolymere, Styrol-Butadien-Blockcopolymere, Isopren-Blockcopolymere, Acrylnitril-Butadien-Kautschuk, Butylkautschuk oder Neoprenkautschuk, sowie Heißschmelzkleber. Als Haftkleber kommen auch solche auf Silikonbasis in Betracht. Mit Vorteil können auch geeignete Mischungen der genannten Polymere zum Einsatz kommen.
Unter den Begriff "Heißschmelzkleber" fallen alle Kleber, die nicht durch Lösemittel, sondern durch Schmelzen bei erhöhten Temperaturen, beispielsweise im Bereich von 60-200 °C verflüssigt werden. Als Heißschmelzkleber eignen sich z. B. Mischungen aus Estern des hydrierten Kolophoniums mit Cellulosederivaten.
Neben den genannten Polymeren können auch weitere, dem Fachmann bekannte Polymere als Grundpolymere für die Herstellung der Matrix oder der Haftklebeschicht verwendet werden, vorausgesetzt, diese sind mit dem Wirkstoff Venlafaxin kompatibel.

Eine besonders bevorzugte Ausführungsform der Erfindung ist dadurch gekennzeichnet, daß der Wirkstoff Venlafaxin im TTS in Kombination mit einem Lösungsvermittler vorliegt, vorzugsweise in gelöstem Zustand; auch ein Gemisch von Lösungsvermittlern kann verwendet werden.
Beispiele für bevorzugte Lösungsvermittler sind mehrwertige Alkohole wie 1,2-Propandiol, die verschiedenen Butandiole, Glycerin, Polyethylenglykol 400, Tetrahydrofurfurylalkohol, Diethylenglykolmonoethylether, Diethyltoluamid und Monoisopropyliden-glycerin. Besonders bevorzugt wird 1,2-Propandiol verwendet. Einige der genannten Lösungsvermittler, wie z. B. auch das 1,2-Propandiol, können zusätzlich auch permeationsfördernd wirken.
Als vorteilhaft hat sich herausgestellt, daß der Anteil des/der Lösungsvermittler zwischen 1 und 50 Gew.-%, vorzugsweise zwischen 5 und 35 Gew.-% beträgt, bezogen auf das gesamte TTS im Endzustand nach der Herstellung.

Um einen hohen Wirkstoff-Flux durch die Haut zu erreichen, hat es sich insbesondere bei Matrix-Systemen als besonders vorteilhaft erwiesen, wenn der Matrix ein oder mehrere permeationsfördernde Stoffe zugesetzt werden, und zwar in einem Mengenanteil von 0,1 bis 25 Gew.-%, vorzugsweise von 1 bis 10 Gew.-%, jeweils bezogen auf das Gesamtgewicht der Wirkstoffmatrix.
Als permeationsfördernde Stoffe eignen sich vor allem Stoffe aus den Gruppen der Fettalkohole, Fettsäuren, Polyoxyethylenfettalkoholether, Polyoxyethylenfettsäureester, Fettalkoholester und Fettsäureester, insbesondere Sorbitanmonolaurat oder Ester von langkettigen Fettsäuren mit Methyl-, Ethyl- oder Isopropylalkohol, oder Ester von Fettalkoholen mit Essigsäure oder Milchsäure. Auch Stoffe wie Ölsäurediethanolamin kommen in Betracht. Besonders bevorzugt werden Polyoxylaurylether (Brij® ) eingesetzt.

Die Wirkstoffmatrix der erfindungsgemäßen TTS kann gemäß einer besonderen Ausführungsform auch einen zwei- oder mehrschichtigen Aufbau aufweisen, d. h. sie kann aus zwei oder mehreren Matrixschichten bestehen. Dabei können sich die verschiedenen Matrixschichten hinsichtlich ihrer Zusammensetzung oder der Konzentration der darin enthaltenen Bestandteile unterscheiden. Beispielsweise können die verschiedenen Matrixschichten eine unterschiedliche Polymerzusammensetzung aufweisen oder aus unterschiedlichen Haftklebern bestehen. Ferner können die einzelnen Matrixschichten unterschiedliche Konzentrationen an Wirkstoff oder Zusatzstoffen wie permeationsfördernden Mitteln, Lösungsvermittlern und Weichmachern enthalten. In Abhängigkeit von dem beabsichtigten Anwendungszweck können beispielsweise die Konzentrationen dieser Inhaltsstoffe, insbesondere die Wirkstoffkonzentration, in diesen Schichten so eingestellt werden, daß sie von der inneren Schicht zu der hautseitig gelegenen Schicht kleiner oder größer werden, je nachdem, ob eine besondere Langzeitwirkung oder eine besonders starke Initialwirkung angestrebt wird.

Bei weiteren Ausführungsformen der erfindungsgemäßen TTS ist vorgesehen, daß der Wirkstoffmatrix, oder einzelnen Schichten dieser Matrix, übliche Weichmacher in einer Konzentration von bis zu 30 Gew.-% zugesetzt werden, besonders bevorzugt in einer Konzentration von 5-20 Gew.-%, jeweils bezogen auf die Wirkstoffmatrix.
Als Weichmacher können bevorzugt solche aus der Gruppe der Kohlenwasserstoffe, Alkohole, Carbonsäuren, Derivate von Carbonsäuren, Ether, Ester und Amine verwendet werden.

Um eine hohe Freisetzungsrate zu erzielen, wird bevorzugt, eine möglichst hohe Wirkstoffkonzentration in der/den wirkstoffhaltigen Schicht(en) vorzusehen, wobei allerdings zu beachten ist, daß zu hohe Wirkstoffkonzentrationen die physikalische Stabilität der Wirkstoffmatrix beeinträchtigen können. Bei den erfindungsgemäßen TTS werden deshalb Wirkstoffkonzentrationen im Bereich von 0,1 bis 50 Gew.-%, bevorzugt im Bereich von 1 bis 10 Gew.-% angewandt, jeweils bezogen auf die Gesamtmasse der wirkstoffhaltigen Schicht(en).

Um eine Steuerung der Wirkstofffreisetzung zu ermöglichen - sofern dies nicht durch andere Mechanismen bewirkt wird -, kann das Wirkstoffreservoir auf der Abgabeseite auch mit einer Steuermembran versehen werden, welche eine begrenzte Durchlässigkeit für den Wirkstoff aufweist und die Abgabe des Wirkstoffs an die Haut steuert.

Die haftklebende Befestigung der erfindungsgemäßen TTS auf der Haut kann auf verschiedene Arten erfolgen. Beispielsweise kann die wirkstoffhaltige Matrix selbst aus einem Haftkleber bestehen und so die Verbindung zur Haut bewirken, oder es ist eine separate haftklebende Schicht angebracht, welche diese Funktion übernimmt. Insbesondere bei Systemen, welche - wie vorstehend beschrieben - mit einer hautseitigen, nicht klebenden Steuermembran ausgestattet sind, ist es vorteilhaft, die Befestigung auf der Haut durch einen Klebstoffrand zu bewirken, der die Membranfläche, über welche der Wirkstoff an die Haut abgegeben wird, umgibt, jedoch nicht berührt; d. h. der Klebstoffrand steht mit der Steuermembran in keiner Verbindung.

Die Erfindung umfaßt ferner Ausführungsformen, bei denen das Venlafaxin enthaltende Wirkstoffreservoir als beutelförmiges Reservoir gestaltet ist, welches mit einer fließfähigen, hochviskosen, halbfesten oder gel-artigen Matrix gefüllt ist, die den Wirkstoff enthält. Beispielsweise kann es sich dabei um eine Polymermatrix, insbesondere um eine Kunststoffmatrix oder eine Lösung davon handeln. Besonders vorteilhaft ist es, wenn das Wirkstoffreservoir einen Gelbildner enthält.
Die der Haut abgewandte Beutelrückseite muß dabei wirkstoffundurchlässig, die der Haut zugewandte Seite (Abgabeseite) wirkstoffdurchlässig sein. Falls erforderlich, kann eine wirkstoffdurchlässige Membran die Steuerung der Wirkstofffreisetzung übernehmen. Geeignete Materialien für die Herstellung der Beutelwand bzw. der Steuermembran sind dem Fachmann bekannt.

Die erfindungsgemäßen TTS weisen neben dem Wirkstoffreservoir außerdem eine wirkstoffundurchlässige Rückschicht sowie eine ebenfalls wirkstoffundurchlässige ablösbare Schutzschicht oder Abziehfolie auf.
Als Materialien für die Rückschicht eignen sich vor allem Polyester, die sich durch besondere Festigkeit auszeichnen, wie z. B. Polyethylenterephthalat und Polybutylenterephthalat, darüber hinaus aber nahezu beliebige andere hautverträgliche Kunststoffe, wie Polyvinylchlorid, Ethylen-Vinylacetat-Copolymere, Polyvinylacetat, Polyethylen, Polypropylen, Polyurethane, Cellulosederivate und viele andere mehr. Im Einzelfall kann die Rückschicht mit einer zusätzlichen Auflage versehen werden, z. B. durch Bedampfung mit Metallen, insbesondere Aluminium.

Für die ablösbare Schutzschicht können grundsätzlich dieselben Materialien verwendet werden wie für die Rückschicht, vorausgesetzt, daß sie durch eine geeignete Oberflächenbehandlung, wie z. B. Silikonisierung, ablösbar ausgerüstet ist. Es können aber auch andere ablösbaren Schutzschichten wie z. B. mit Polytetrafluorethylen behandeltes Papier oder Cellophan® (Cellulosehydrat) verwendet werden.

Die erfindungsgemäßen TTS mit dem Wirkstoff Venlafaxin eignen sich in vorteilhafter Weise zur Prophylaxe und Therapie von Psychosen, insbesondere Depressionen, sowie von Angstzuständen, Neurosen und Psychopathien. Besonders vorteilhaft ist dabei, daß mit diesen Darreichungsformen eine Wirkungsdauer von mindestens etwa einem Tag bis zu etwa 7 Tagen erreicht werden kann, abhängig vom Wirkstoff-Gehalt und der Ausgestaltung des Steuerungsmechanismus, der das Wirkstoffabgabeverhalten kontrolliert.

Gemäß einer weiteren Ausführungsform der Erfindung ist vorgesehen, daß das Wirkstoffreservoir Venlafaxin in Kombination mit mindestens einem weiteren pharmazeutischen Wirkstoff enthält; vorzugsweise handelt es sich dabei um einen zentral wirksamen Arzneistoff.

Die Erfindung wird durch nachfolgendes Beispiel erläutert.

### Beispiel

Die erfindungsgemäßen TTS können beispielsweise wie folgt hergestellt werden:

Es werden 50 g Venlafaxin sowie 20 g eines geeigneten permeationsfördernden Stoffes (z. B. Brij® 30) in 200 g 1,2-Propandiol gelöst. Diese Lösung wird mit einer geeigneten Rührapparatur in einen Silikonkleber (Nr. 4301; Fa. Dow Corning, USA) gegeben und dispergiert, so daß eine möglichst homogene Flüssig-Flüssig-Dispersion entsteht. Diese Dispersion wird mit einer geeigneten Vorrichtung homogen auf eine Trägerfolie, z. B. aus Polyethylenterephthalat beschichtet. Anschließend wird durch eine kontrollierte Trocknung das Lösungsmittel des Silikonklebers sowie etwaige Anteile des Propandiols entfernt. Kontrollierte Trocknung bedeutet, daß das beschichtete Laminat einer ganz bestimmten Trocknungstemperatur, Trocknungsgeschwindigkeit oder Trocknungszeit ausgesetzt wird, um den beabsichtigten Gehalt von flüchtigen Substanzen (z. B. Lösungsvermittler) einzustellen.
Das so erhaltene Laminat wird anschließend mit einer weiteren Folie aus Polyethylenterephthalat zukaschiert. Zuletzt werden TTS mit einer bestimmten Fläche ausgestanzt und in ein geeignetes Packmittel verpackt.

## Patentansprüche

1. Transdermales therapeutisches System in Pflasterform zur Verabreichung des Wirkstoffs Venlafaxin, welches eine wirkstoffundurchlässige Rückschicht, ein damit verbundenes, den Wirkstoff Venlafaxin enthaltendes Wirkstoffreservoir, eine hautseitige haftklebende Schicht und eine vor der Applikation ablösbare wirkstoffundurchlässige Schutzschicht aufweist.

2. Transdermales therapeutisches System nach Anspruch 1, **dadurch gekennzeichnet, daß** das Wirkstoffreservoir als wirkstoffhaltige Matrix ausgebildet ist, wobei die genannte Matrix eine Kunstharz- oder Kunststoffmatrix ist, die als Grundpolymer(e) ein oder mehrere Polymere enthält, welche vorzugsweise aus der Polyacrylate, Poly(meth)acrylate, Polyacrylsäure, Cellulose-Derivate, Isobutylen, Ethylen-Vinylacetat, natürliche und synthetische Kautschuke wie Styrol-Dien-Copolymere, Styrol-Butadien-Blockcopolymere, Isopren-Blockcopolymere, Acrylnitril-Butadien-Kautschuk, Butylkautschuk oder Neoprenkautschuk, sowie Heißschmelzkleber umfassenden Gruppe ausgewählt sind.

3. Transdermales therapeutisches System nach Anspruch 1 oder 2, **dadurch gekennzeichnet, daß** das Wirkstoffreservoir als wirkstoffhaltige Matrix ausgebildet ist, in welcher der Wirkstoff Venlafaxin an einem Fasermaterial, bevorzugt an Baumwollgewebe oder -vlies adsorbiert vorliegt, wobei das genannte Fasermaterial vorzugsweise in eine Kunststoff-oder Kunstharzmatrix eingebettet ist.

4. Transdermales therapeutisches System nach Anspruch 1, **dadurch gekennzeichnet, daß** das Wirkstoffreservoir als beutelförmiges Reservoir ausgebildet ist, welches eine hochviskose oder halbfeste Kunststoffmatrix oder eine Lösung davon oder einen Gelbildner oder ein Gel enthält, worin der Wirkstoff Venlafaxin gelöst oder dispergiert ist.

5. Transdermales therapeutisches System nach einem oder mehreren der Ansprüche 1 bis 4, **dadurch gekennzeichnet, daß** das Wirkstoffreservoir hautseitig mit einer wirkstoffdurchlässigen oder einer die Wirkstoffabgaberate beschränkenden Steuermembran ausgestattet ist.

6. Transdermales therapeutisches System nach Anspruch 5, **dadurch gekennzeichnet, daß** es einen an den Außenrand der Membranfläche angrenzenden, auf der Haut haftklebenden Klebstoffrand besitzt, der nicht mit der Steuermembran in Verbindung steht.

7. Transdermales therapeutisches System nach einem oder mehreren der vorangehenden Ansprüche, **dadurch gekennzeichnet, daß** die haftklebende Schicht als Grundpolymere Polymere enthält, welche vorzugsweise aus der Polyacrylate, Poly-(meth)acrylate, Polyacrylsäure, Cellulose-Derivate, Isobutylen, Ethylen-Vinylacetat, natürliche und synthetische Kautschuke wie Styrol-Dien-Copolymere, Styrol-Butadien-Blockcopolymere, Isopren-Blockcopolymere, Acrylnitril-Butadien-Kautschuk, Butylkautschuk oder Neoprenkautschuk, sowie Heißschmelzkleber und Haftkleber auf Silikonbasis umfassenden Gruppe ausgewählt sind.

8. Transdermales therapeutisches System nach einem oder mehreren der vorangehenden Ansprüche, **dadurch gekennzeichnet, daß** das Wirkstoffreservoir als haftklebende Schicht ausgebildet ist.

9. Transdermales therapeutisches System nach einem oder mehreren der vorangehenden Ansprüche, **dadurch gekennzeichnet, daß** das Wirkstoffreservoir und/oder die haftklebende Schicht mindestens einen Hilfs- oder Zusatzstoff enthält, welcher aus der Lösemittel, Lösungsvermittler, Weichmacher, Permeationsverbesserer, pH-Regulatoren, Antioxidantien und Konservierungmittel umfassenden Gruppe ausgewählt ist.

10. Transdermales therapeutisches System nach einem oder mehreren der vorangehenden Ansprüche, **dadurch gekennzeichnet, daß** es den Wirkstoff Venlafaxin in Kombination mit einem Lösungsvermittler, besonders bevorzugt einem Lösungsvermittler mit permeationsfördernder Wirkung, enthält, wobei der Wirkstoff vorzugsweise in gelöster Form vorliegt.

11. Transdermales therapeutisches System nach einem oder mehreren der vorangehenden Ansprüche, **dadurch gekennzeichnet, daß** es einen oder mehrere Lösungsvermittler enthält, vorzugsweise ausgewählt aus der mehrwertige Alkohole, 1,2-Propandiol, Butandiole, Glycerin, Polyethylenglykol 400, Tetrahydrofurfurylalkohol, Diethylenglykolmonoethylether, Diethyltoluamid und Monoisopropyliden-glycerin umfassenden Gruppe, wobei die Konzentration des/der Lösungsvermittlers zwischen 1 und 50 Gew.-%, vorzugsweise zwischen 5 und 35 Gew.-% beträgt, bezogen auf das gesamte System.

12. Transdermales therapeutisches System nach einem oder mehreren der vorangehenden Ansprüche, **dadurch gekennzeichnet, daß** es einen oder mehrere permeationsfördernde Stoffe enthält, vorzugsweise in einer Konzentration von 0,1 bis 25 Gew.-%, besonders bevorzugt in einer Konzentration von 1 bis 10 Gew.-%, jeweils bezogen auf das gesamte System.

13. Transdermales therapeutisches System nach Anspruch 12, **dadurch gekennzeichnet, daß** der/die permeationsfördernde(n) Stoff(e) aus der Fettalkohole, Fettsäuren, Polyoxyethylenfettalkoholether, Polyoxyethylenfettsäureester, Fettsäureester und Fettalkoholester umfassenden Gruppe ausgewählt ist.

14. Transdermales therapeutisches System nach einem oder mehreren der vorangehenden Ansprüche, **dadurch gekennzeichnet, daß** die Wirkstoffmatrix einen oder mehrere Weichmacher enthält, vorzugsweise in einer Konzentration von bis zu 30 Gew.-%, besonders bevorzugt in einer Konzentration von 5-20 Gew.-%, jeweils bezogen auf die Wirkstoffmatrix, wobei die Weichmacher vorzugsweise aus der Kohlenwasserstoffe, Alkohole, Carbonsäuren, Derivate von Carbonsäuren, Ether, Ester und Amine umfassenden Gruppe ausgewählt sind.

15. Transdermales therapeutisches System nach einem oder mehreren der vorangehenden Ansprüche, **dadurch gekennzeichnet, daß** das Wirkstoffreservoir einen schichtförmigen Aufbau mit zwei oder mehreren Matrixschichten aufweist, die sich hinsichtlich ihres Polymergehalts und/oder ihres Wirkstoffgehalts und/oder hinsichtlich der Art und Konzentration der darin enthaltenen Permeationsverbesserer, Weichmacher oder Lösungsvermittler unterscheiden.

16. Transdermales therapeutisches System nach einem oder mehreren der vorangehenden Ansprüche, **dadurch gekennzeichnet, daß** die Wirkstoffkonzentration im Bereich von 0,1 bis 50 Gew.-%, bevorzugt im Bereich von 1 bis 10 Gew.-% liegt, jeweils bezogen auf die Gesamtmasse der wirkstoffhaltigen Schicht (en).

17. Transdermales therapeutisches System nach einem oder mehreren der vorangehenden Ansprüche, **dadurch gekennzeichnet, daß** das Wirkstoffreservoir neben Venlafaxin mindestens einen weiteren Wirkstoff enthält, vorzugsweise einen zentral wirksamen Wirkstoff.

18. Verwendung eines den Wirkstoff Venlafaxin enthaltenden transdermalen therapeutischen Systems zur Herstellung eines Medikaments zur Prophylaxe und Therapie von Psychosen, insbesondere Depressionen, sowie von Angstzuständen, Neurosen und Psychopathien.

19. Verwendung eines transdermalen therapeutischen Systems nach einem der Ansprüche 1 bis 17 zur zur Herstellung eines Medikaments Prophylaxe und Therapie von Psychosen, insbesondere Depressionen, sowie von Angstzuständen, Neurosen und Psychopathien.

20. Verwendung von Venlafaxin zur Herstellung eines transdermalen therapeutischen Systems zur prophylaktischen oder therapeutischen Behandlung von Patienten, die an Psychosen, insbesondere Depressionen, oder Angstzuständen, Neurosen oder Psychopathien leiden.

## Claims

1. Transdermal therapeutic system in patch form for administering the active agent venlafaxine, which comprises an active agent-impermeable backing layer, an active agent reservoir connected therewith and containing the active agent venlafaxine, a pressure-sensitive adhesive layer on the side of the skin, and an active agent-impermeable protective layer detachable prior to application.

2. Transdermal therapeutic system according to claim 1, **characterized in that** the active agent reservoir is formed as an active agent-containing matrix, the said matrix being a synthetic resin or plastics matrix containing as base polymer(s) one or more polymers which are preferably selected from the group comprising the polyacrylates, poly(meth)acrylates, polyacrylic acids, cellulose derivatives, isobutylene, ethylene vinyl acetate, natural and synthetic rubbers such as styrene-diene copolymers, styrene-butadiene block copolymers, isoprene block copolymers, acrylonitrile-butadiene rubber, butyl rubber or neoprene rubber, as well as hot-melt adhesives.

3. Transdermal therapeutic system according to claim 1 or 2, **characterized in that** the active substance reservoir is formed as active agent-containing matrix wherein the active agent venlafaxine is present adsorbed on a fibre material, preferably on cotton fabric or nonwoven, said fibre material preferably being embedded in a plastics or a synthetic resin matrix.

4. Transdermal therapeutic system according claim 1, **characterized in that** the active substance reservoir is formed as a pouch-shaped reservoir containing a high-viscous or semi-solid plastics matrix or a solution thereof, or a gelatinizing agent, or a gel, wherein the active substance venlafaxine is dissolved or dispersed.

5. Transdermal therapeutic system according to one or more of claims 1 to 4, **characterized in that** the active substance reservoir is provided on the skin-side with an active substance-permeable control membrane or with a control membrane limiting the active agent release rate.

6. Transdermal therapeutic system according to claim 5, **characterized in that** it comprises an adhesive margin which is adjacent to the outer edge of the membrane surface, is pressure-sensitive adhesive on the skin, and which is not in contact with the control membrane.

7. Transdermal therapeutic system according to one or more of the preceding claims, **characterized in that** the pressure-sensitive adhesive layer contains, as base polymers, polymers which are preferably selected from the group comprising polyacrylates, poly(meth)acrylates, polyacrylic acid, cellulose derivatives, isobutylene, ethylene vinyl acetate, natural and synthetic rubbers such as styrene-diene copolymers, styrene-butadiene block copolymers, isoprene block copolymers, acrylonitrile-butadiene rubber, butyl rubber or neoprene rubber, as well as hot-melt adhesives and silicone-based pressure-sensitive adhesive.

8. Transdermal therapeutic system according to one or more of the preceding claims, **characterized in that** the active agent reservoir is formed as a pressure-sensitive adhesive layer.

9. Transdermal therapeutic system according to one or more of the preceding claims **characterized in that** the active agent reservoir and/or the pressure-sensitive adhesive layer contains at least one auxiliary or additive substance which is selected from the group comprising solvents, solubilizers, plasticizers, permeation enhancers, pH-regulators, antioxidants and preservatives.

10. Transdermal therapeutic system according to one or more of the preceding claims, **characterized in that** it contains the active agent venlafaxine in combination with a solubilizer, especially preferred a solubilizer with permeation-enhancing action, said active agent preferably being present in dissolved form.

11. Transdermal therapeutic system according to one or more of the preceding claims, **characterized in that** it contains one or more solubilizers, preferably selected from the group comprising polyvalent alcohols, 1,2-propanediol, butanediol, glycerol, polyethylene glycol 400, tetrahydrofurfuryl alcohol, diethylene glycol monoethyl ether, diethyl toluamide and monoisopropylidene glycerol, the concentration of the solubilizer(s) being between 1 and 50%-wt., preferably between 5 and 35%-wt., relative to the total system.

12. Transdermal therapeutic system according to one or more of the preceding claims, **characterized in that** it contains one or more permeation-enhancing substances, preferably in a concentration of 0.1 to 25%-wt., especially preferred in a concentration of 1 to 10%-wt., in each case relative to the total system.

13. Transdermal therapeutic system according to claim 12, **characterized in that** the permeation-enhancing substances are selected from the group comprising fatty alcohols, fatty acids, polyoxyethylene fatty alcohol ethers, polyoxyethylene fatty acid esters, fatty acid esters and fatty alcohol esters.

14. Transdermal therapeutic system according to one or more of the preceding claims, **characterized in that** the active agent matrix contains one or more plastics, preferably in a concentration of up to 30%-wt, especially preferred in a concentration of 5-20%-wt., in each case relative to the active substance matrix, said softeners preferably being selected from the group comprising carbohydrates, alcohols, carboxylic acids, derivatives of carboxylic acids, ethers, esters and amines.

15. Transdermal therapeutic system according to one or more of the preceding claims, **characterized in that** the active agent reservoir has a laminar structure with two or more matrix layers which differ in terms of their polymer content and/or their active agent content and/or in terms of the kind and concentration of the permeation enhancers, plasticizers or solubilizers contained therein.

16. Transdermal therapeutic system according to one or more of the preceding claims, **characterized in that** the active agent concentration is in the range of 0.1 to 50%-wt., preferably in the range of 1 to 10%-wt., in each case relative to the total mass of the active agent-containing layer(s).

17. Transdermal therapeutic system according to one or more of the preceding claims, **characterized in that** the active agent reservoir contains, apart from venlafaxine, at least one further active agent, preferably a centrally active agent.

18. Use of a transdermal therapeutic system containing the active agent venlafaxine for the production of a medicament intended for the prophylaxis and therapy of psychoses, especially depressions, as well as of anxiety states, neuroses and psychopathies.

19. Use of a transdermal therapeutic system according to any one of claims 1 to 17 for the production of a medicament intended for the prophylaxis and therapy of psychoses, especially depressions, as well as of anxiety states, neuroses and psychopathies.

20. Use of venlafaxine for the production of a transdermal therapeutic system for the prophylactic or therapeutic treatment of patients suffering from psychoses, especially depressions, or anxiety states, neuroses or psychopathies.

## Revendications

1. Système thérapeutique transdermique sous forme d'emplâtre aux fins d'administration de la substance active venlafaxine, qui présente une couche dorsale imperméable à la substance active, un réservoir de substance active qui y est relié, contenant la substance active venlafaxine, une couche adhésive par contact du côté de la peau et une couche protectrice imperméable à la substance active qui peut être détachée avant application.

2. Système thérapeutique transdermique selon la revendication 1, **caractérisé en ce que** le réservoir de substance active est formé en matrice contenant une substance active, la matrice en question étant une matrice de résine synthétique ou de matière plastique qui contient comme polymère(s) de base un ou plusieurs polymères qui sont choisis de préférence dans le groupe qui comprend les polyacrylates, les poly(méth)acrylates, l'acide polyacrylique, les dérivés de cellulose, l'isobutylène, l'éthylène-acétate de vinyle, les caoutchoucs naturels et synthétiques comme les copolymères styrène-diène, les copolymères séquencés styrène-butadiène, les copolymères séquencés d'isoprène, le caoutchouc acrylonitrile-butadiène, le caoutchouc butyle ou le caoutchouc néoprène, ainsi que les colles thermofusibles.

3. Système thérapeutique transdermique selon la revendication 1 ou 2, **caractérisé en ce que** le réservoir de substance active est formé en matrice contenant une substance active, dans laquelle la substance active venlafaxine se présente adsorbée sur un matériau fibreux, de préférence sur un tissu ou un non-tissé de coton, le matériau fibreux mentionné étant de préférence enrobé dans une matrice de matière plastique ou de résine synthétique.

4. Système thérapeutique transdermique selon la revendication 1, **caractérisé en ce que** le réservoir de substance active est formé en réservoir en forme de pochette qui contient une matrice de matériau synthétique hautement visqueuse ou semi-solide ou une solution de ce matériau, ou un agent gélifiant ou un gel, la substance active venlafaxine y étant dissoute ou dispersée.

5. Système thérapeutique transdermique selon une ou plusieurs des revendications 1 à 4, **caractérisé en ce que** le réservoir de substance active est équipé du côté de la peau d'une membrane de régulation perméable à la substance active ou limitant la vitesse de libération de la substance active.

6. Système thérapeutique transdermique selon la revendication 5, **caractérisé en ce qu'**il possède un bord adhésif adhérant à la peau, avoisinant le bord extérieur de la surface de la membrane, qui n'est pas en liaison avec la membrane de régulation.

7. Système thérapeutique transdermique selon une ou plusieurs des revendications précédentes, **caractérisé en ce que** la couche adhérant par contact contient comme polymères de base des polymères qui sont de préférence choisis dans le groupe comprenant les polyacrylates, les poly(méth)acrylates, l'acide polyacrylique, les dérivés de cellulose, l'isobutylène, l'éthylène-acétate de vinyle, les caoutchoucs naturels et synthétiques comme les copolymères styrène-diène, les copolymères séquencés styrène-butadiène, les copolymères séquencés d'isoprène, le caoutchouc acrylonitrile-butadiène, le caoutchouc butyle ou le caoutchouc néoprène, ainsi que des adhésifs thermofusibles et des adhésifs par contact à base de silicone.

8. Système thérapeutique transdermique selon une ou plusieurs des revendications précédentes, **caractérisé en ce que** le réservoir de substance active est formé en couche adhérant par contact.

9. Système thérapeutique transdermique selon une ou plusieurs des revendications précédentes, **caractérisé en ce que** le réservoir de substance active et/ou la couche adhérant par contact contient au moins un adjuvant ou additif qui est choisi parmi les solvants, les agents de solubilisation, les plastifiants, les agents améliorant la perméation, les régulateurs de pH, les antioxydants et les agents de conservation.

10. Système thérapeutique transdermique selon une ou plusieurs des revendications précédentes, **caractérisé en ce qu'**il contient la substance active venlafaxine en combinaison avec un agent de solubilisation, de façon particulièrement préférée un agent de solubilisation ayant un effet favorisant la perméation, la substance active se présentant de préférence sous forme dissoute.

11. Système thérapeutique transdermique selon une ou plusieurs des revendications précédentes, **caractérisé en ce qu'**il contient un ou plusieurs agents de solubilisation, de préférence choisis parmi les alcools polyvalents, le 1,2-propanediol, les butanediols, la glycérine, le polyéthylèneglycol 400, l'alcool tétrahydrofurfurylique, l'éther monoéthylique de diéthylèneglycol, le diéthyltoluamide et la monoisopropylidène-glycérine, la concentration du ou des agents de solubilisation se situant entre 1 et 50 % en poids, de préférence entre 5 et 35 % en poids, par rapport à l'ensemble du système.

12. Système thérapeutique transdermique selon une ou plusieurs des revendications précédentes, **caractérisé en ce qu'**il contient une ou plusieurs substances favorisant la perméation, de préférence en une concentration de 0,1 à 25 % en poids, de façon particulièrement préférée en une concentration de 1 à 10 % en poids, toujours par rapport à l'ensemble du système.

13. Système thérapeutique transdermique selon la revendication 12, **caractérisé en ce que** la ou les substances favorisant la perméation sont choisies parmi les alcools gras, les acides gras, les éthers d'alcools gras de polyoxyéthylène, les esters d'acides gras de polyoxyéthylène, les esters d'acides gras et les esters d'alcools gras.

14. Système thérapeutique transdermique selon une ou plusieurs des revendications précédentes, **caractérisé en ce que** la matrice de substance active contient un ou plusieurs plastifiants, de préférence en une concentration allant jusqu'à 30 % en poids, de façon particulièrement préférée en une concentration de 5 à 20 % en poids, toujours par rapport à la matrice de substance active, les plastifiants étant choisis de préférence parmi les hydrocarbures, les alcools, les acides carboxyliques, les dérivés d'acides carboxyliques, les éthers, les esters et les amines.

15. Système thérapeutique transdermique selon une ou plusieurs des revendications précédentes, **caractérisé en ce que** le réservoir de substance active présente une constitution stratifiée avec deux ou plusieurs couches de matrice qui se différencient quant à leur teneur en polymère et/ou leur teneur en substance active et/ou quant au type et à la concentration des améliorateurs de perméation, plastifiants ou agents de solubilisation qui y sont contenus.

16. Système thérapeutique transdermique selon une ou plusieurs des revendications précédentes, **caractérisé en ce que** la concentration de substance active se situe dans un intervalle allant de 0,1 à 50 % en poids, de préférence de 1 à 10 % en poids, toujours par rapport à la masse totale de la ou des couches contenant la substance active.

17. Système thérapeutique transdermique selon une ou plusieurs des revendications précédentes, **caractérisé en ce que** le réservoir de substance active contient, outre la venlafaxine, au moins une autre substance active, de préférence une substance active à effet central.

18. Utilisation d'un système thérapeutique transdermique contenant la substance active venlafaxine pour la fabrication d'un médicament en vue de la prophylaxie et du traitement des psychoses, en particulier des dépressions, ainsi que des états d'angoisse, des névroses et des psychopathies.

19. Utilisation d'un système thérapeutique transdermique selon l'une des revendications 1 à 17 pour la fabrication d'un médicament en vue de la prophylaxie et du traitement des psychoses, en particulier des dépressions, ainsi que des états d'angoisse, des névroses et des psychopathies.

20. Utilisation de venlafaxine pour la production d'un système thérapeutique transdermique en vue du traitement prophylactique ou thérapeutique de malades qui souffrent de psychoses, en particulier de dépressions, ou d'états d'angoisse, de névroses ou de psychopathies.
